# EUROPÄISCHE PATENTSCHRIFT

(11) **EP 1 644 735 B1**
(45) Veröffentlichungstag und Bekanntmachung des Hinweises auf die Patenterteilung: **21.09.2011**
(21) Anmeldenummer: 04740819.0
(22) Anmeldetag: 08.07.2004
(51) Int. Cl.: G01N 33/543

(54) **VORRICHTUNG FÜR LATERAL-FLUSS-TESTS**
DEVICE FOR LATERAL FLOW TESTS
DISPOSITIF POUR TESTS SUR MEMBRANE A ECOULEMENT LATERAL (LATERAL FLOW)

(30) Priorität: 09.07.2003 DE 10330983
(43) Veröffentlichungstag der Anmeldung: 12.04.2006
(73) Patentinhaber: Medion Diagnostics AG, 3186 Düdingen (CH)
(72) Erfinder: SCHWIND, Peter, CH-1700 Fribourg (CH); LÖSTER, Klemens, 16562 Bergfelde (DE)
(74) Vertreter: Bublak, Wolfgang
(86) Internationale Anmeldenummer: PCT/EP2004/007526
(87) Internationale Veröffentlichungsnummer: WO 2005/005966

(56) Entgegenhaltungen:
- WO-A-99/09410
- WO-A-03/012443
- WO-A-03/104490
- WO-A1-97/32213

## Beschreibung

Die Erfindung betrifft eine Vorrichtung für Lateral-Diagonal-Fluss Multiparameter Tests für biologische oder nicht-biologische Testsysteme zum gleichzeitigen und qualitativen oder quantitativen Bestimmen mehrerer zellulär-gebundener und plasmatischer Analyten, wobei wenigstens ein Analyt ein zellulär-gebundener Analyt ist, in einer flüssigen Probe, umfassend mindestens eine Membran mit einer Aufgabezone zum Auftragen der flüssigen Probe, mindestens einer Gruppe von mindestens zwei Indikatorzonen, die mit den Analyten in Wechselwirkung treten können und mindestens einem Absorptionsbereich, welcher die Flüssigkeit nach Passieren der Indikatorzonen aufnimmt, wobei die Indikatorzonen zwischen der Aufgabezone und einem Absorptionsbereich liegen, dadurch gekennzeichnet, dass die Fließrichtungen von der Aufgabezone durch die jeweiligen Indikatorzonen einer Gruppe zu einem Absorptionsbereich (Fließspuren) im Wesentlichen parallel sind und mindestens zwei unterschiedliche Fließspuren vorliegen und sofern die Indikatorzonen mit mehr als einem zellulär-gebundenen Analyten reagieren, die Indikatorzonen für diese Analyten nicht hintereinander angeordnet sind und wobei die Indikatorzonen so angeordnet sind, dass die Indikatorzone des zellulär-gebundenen Analyten distal zur Aufgabezone und die Indikatorzone des plasmatischen Analyten proximal zur Aufgabezone angeordnet sind.

Lateral-Fluss-Tests finden heute vielfach Anwendung als Schnelltests, z. B. als Schwangerschaftstests, zur Bestimmung von Infektionsmarkern oder als Drogenscreen. Eine Lateral-Fluss-Test-Anordnung besteht bekanntermaßen aus einem festen Träger, auf dem eine Aufgabezone für die zu untersuchende Probe aufgebracht ist, eine Trennmembran, auf der Bindungselemente, z. B. Fängerantikörper bzw. -antigene gebunden sind und auf der sich Bindungsreaktionen nachweisen lassen und ein saugfähiger Absorptionsbereich, der die zu untersuchende Probe durch die Trennmembran fließen lässt.

WO 03/012443 betrifft ein Membran -Testsystem mit "Zwei-Schritt Test" und umfasst eine Test-Einheit und eine Post-Filter-Einheit.

WO 99/09410 betrifft einen Assay zum gleichzeitigen Bestimmen der Analyten HIV-1 Group M, HIV-1 Group O und HIV-2 unter Verwendung der sequenzspezifische Polypeptide.

WO 97/32213 beschreibt eine Vorrichtung zum Bestimmen verschiedenen Blutgruppen. Die Vorrichtung umfasst ein Membranblatt beschichtet mit Antibody und ein Absorptionsauflager.

Testmembranen herkömmlicher Lateral-Fluss-Tests werden in der Regel mit chromatographie-ähnlicher Auftrennung beschrieben. Der Analyt in der Probe bindet spezifisch an die in einer Membran befestigten Bindungselemente, die in der Regel in hintereinanderliegenden bzw. übereinanderstehenden Banden angeordnet als Indikatorzonen vorliegen. Der Bindungskomplex wird durch Indikatorpartikel sichtbar gemacht, welche in der Regel in einem Konjugat-Freisetzungs-Pad eingetrocknet in der Anordnung bereits vorliegen. Das Konjugat-Freisetzungs-Pad ist typischerweise zwischen Aufgabezone und Membran angebracht. Die vorbeschichteten farbigen Indikatorpartikel sind beispielsweise mit einem gegen den gesuchten Analyten gerichteten Antikörper beschichtet.

Das übliche Lateral-Fluss-Test Format ist das eines sogenannten "Sandwich-Assays", bei dem sowohl die Indikatorzone als auch die Indikatorpartikel mit gegen den gesuchten Analyten gerichteten Liganden, normalerweise Antikörpern, belegt sind. Dabei ist der Ligand (Bindungselement) an die Membran immobilisiert. Das Detektor-Reagenz, normalerweise ein Antikörper, welcher an gefärbte Polystyrolpartikel oder kolloidale Metalle gebunden ist, ist im Konjugat-Freisetzungs-Pad auswaschbar deponiert. Dieser Bindungskomplex dient als Indikatorpartikel. Nach Auftragen der zu untersuchenden Probe benetzt diese sehr schnell das Konjugat-Freisetzungs-Pad, wodurch die Indikatorpartikel mobilisiert werden. Die Indikatorpartikel migrieren mit der Flüssigkeitsfront entlang der porösen Membran. Ein in der Probe befindlicher Analyt wird durch den Antikörper, der an das Indikatorpartikel gekoppelt ist, gebunden. Wenn die Probe die Indikatorzone passiert, wird der Analyt/Indikatorpartikel-Komplex in der Indikatorzone durch Reaktion des Analyten mit dem in der Indikatorzone gebundenen Antikörper immobilisiert, was zu einem sichtbaren Signal führt.

Ein weiteres bekanntes Testformat für kleine Analyten mit nur einer einzigen antigenen Determinante, die nicht gleichzeitig zwei Antikörper binden kann, ist der sogenannte "Kompetitions-Assay". Das an die Indikatorpartikel gebundene Detektor-Reagenz ist normalerweise ein dem Analyten identisches oder analoges Molekül. Die Indikatorpartikel sind im Konjugat-Freisetzungs-Pad deponiert. Die Indikatorpartikel migrieren mit der Flüssigkeitsfront entlang der porösen Membran. Wenn die Probe, die Analyt enthält, und die Indikatorpartikel (die effektiv ebenfalls Analyt enthalten) die Indikatorzone passieren, bindet ein Teil der Analytmoleküle in der Probe und ein Teil der Indikatorpartikel an die Bindungselemente. Je mehr Analyt sich in der Probe befindet, umso effektiver wird er mit der Bindung der Indikatorpartikel kompetieren, umso schwächer wird das Signal. Bekanntermaßen sind diese Indikatorpartikel überwiegend aus kolloidalem Gold oder aus Polystyrol, die mit dem Fachmann bekannten Methoden hergestellt und beschichtet werden. In den typischen Lateral-Fluss-Tests Formaten werden die Analyten indirekt bestimmt. Unter direkter Bestimmung eines Analyten wird hier verstanden, dass der Analyt bereits an das Indikatorpartikel (z. B. Erythrozyt) natürlich gebunden ist. In dem gebräuchlicheren Fall der indirekten Bestimmung des Analyten enthält die zu testende Probe in der Regel eine nicht zellulär gebundene, z. B. plasmatische Komponente als Analyten und es werden neben der zu testenden Probe zwei Reagenz-Komponenten benötigt, nämlich Indikatorpartikel und Bindungselement. Bei der indirekten Bestimmung bindet der Analyt zunächst an die aus dem Konjugat-Freisetzungs-Pad herausgelösten Indikator-Partikel, bevor dieser Komplex dann durch eine zweite Reaktion mit dem Bindungselement in den Indikatorzonen immobilisiert wird.

In der blutgruppenserologischen Diagnostik werden allgemein Parameter nachgewiesen, die besonders im Zusammenhang mit Transfusionen bzw. dem Morbus Hämolyticus Neonatorum von Bedeutung sind. Dabei handelt es sich unter anderem um den Nachweis von Antigenen auf der Oberfläche der Erythrozyten, die für die Blutgruppen charakteristisch sind. Weitere wichtige Antigensysteme befinden sich auch auf Thrombozyten, Granulozyten, Lymphozyten, die ebenfalls bei Transfusion und/oder Transplantation eine Rolle spielen.

Bei der Verwendung herkömmlicher Lateral-Fluss-Tests mit Erythrozyten als Indikatorpartikeln, die die zu bestimmenden Analyten, beispielsweise Blutgruppen-spezifische Antigene, gebunden haben, werden bislang in den Indikatorzonen Antikörper gegen korrespondierende Blutgruppenantigene als Bindungselemente in hintereinanderliegenden bzw. übereinanderstehenden Banden nur einer Fließspur angeordnet, wie zum Beispiel anti-A, anti-B gegen die Blutgruppen-Antigene A bzw. B oder Antikörper gegen Antigene des Rh Blutgruppensystems. Dabei weisen herkömmliche Lateral-Fluss-Tests den Nachteil auf, dass die an die Antikörper gebundenen Erythrozyten eine Flussbarriere für die weiter zu untersuchenden Analyte, beispielsweise weitere Zell-assoziierte Antigene, in einer Probe bilden. Durch Agglutination oder Adsorption von Zellen in einer proximal zur Aufgabezone liegenden Bande von Bindungselementen können sich weitere Analyten, insbesondere assoziiert an Zellen bzw. Zellfragmente, in der zu untersuchenden Probe nicht weiter ungehemmt und sichtbar auftrennen und können folglich nicht eindeutig bzw. vollständig nachgewiesen werden. Dies kann z. B. bei einer Person, die Blutgruppe AB Rh D positiv ist, zu einer Abschwächung bzw. Eliminierung der B- und der D-Bande führten, was zu einer Fehlinterpretation im Sinne von Blutgruppe A Rh negativ führten könnte. Bislang konnten deshalb speziell in der blutgruppenserologischen Diagnostik keine Lateral-Fluss-Tests mit mehr als einer Indikatorzone angewendet werden. Für die Messung mehrerer, insbesondere zellulärer und plasmatischer, Blutgruppenparameter müssen bislang Einzelparameter-Tests separat durchgerührt werden.

Aufgabe der Erfindung ist es, die im Hinblick auf den Stand der Technik angeführten Nachteile, insbesondere die der hintereinanderliegenden bzw. überlagernden Indikator- bzw. Nachweiszonen herkömmlicher Lateral-Fluss-Tests, für eine gleichzeitige Messung verschiedener Proben-Parameter, d.h. von zellulär gebundenen und plasmatischen Parametern, zu überwinden.

Die Aufgabe wird erfindungsgemäß gelöst durch eine Vorrichtung zum gleichzeitigen und qualitativen oder quantitativen Bestimmen mehrerer zellulär-gebundener und plasmatischer Analyten, wobei wenigstens ein Analyt ein zellulär-gebundener Analyt ist, in einer flüssigen Probe, umfassend mindestens eine Membran mit einer Aufgabezone zum Auftragen der flüssigen Probe, mindestens einer Gruppe von mindestens zwei Indikatorzonen, die mit den Analyten in Wechselwirkung treten können und mindestens einem Absorptionsbereich, welcher die Flüssigkeit nach Passieren der Indikatorzonen aufnimmt, wobei die Indikatorzonen zwischen der Aufgabezone und einem Absorptionsbereich liegen, dadurch gekennzeichnet, dass die Fließrichtungen von der Aufgabezone durch die jeweiligen Indikatorzonen einer Gruppe zu einem Absorptionsbereich (Fließspuren) im Wesentlichen parallel sind und mindestens zwei unterschiedliche Fließspuren vorliegen und sofern die Indikatorzonen mit mehr als einem zellulär-gebundenen Analyten reagieren, die Indikatorzonen für diese Analyten nicht hintereinander angeordnet sind und wobei die Indikatorzonen so angeordnet sind, dass die Indikatorzone des zellulär-gebundenen Analyten distal zur Aufgabezone und die Indikatorzone des plasmatischen Analyten proximal zur Aufgabezone angeordnet sind. Die Indikatorzonen der erfindungsgemäßen Vorrichtung befinden sich auf der Membran und umfassen Bindungselemente, die den/die zu bestimmenden Analyten/Analyte in der/den Proben abfangen bzw. binden. In den Indikatorzonen werden die Bindungsreaktionen zwischen Analyt und/oder Indikatorpartikel und Bindungselement nachgewiesen. Als besonders bevorzugte Bindungselemente werden Antikörper bzw. Antikörperfragmente, Lektine, Antigene bzw. Antigenepitope und/oder Zellen bzw. Zellfragmente an der porösen Membran angebracht.

Es werden insbesondere zwei Gruppen von Indikatorpartikeln verwendet. Die eine wird alleine durch Erythrozyten zum direkten Nachweis von Erythrozyten-gebundenen Analyten repräsentiert. Die andere Gruppe besteht aus Partikeln jeder denkbaren Art und Kombination, mit denen sich Bindungsreaktionen nachweisen lassen, vorzugsweise Partikel aus kolloidalem Gold oder aus Polystyrol oder fixierte Erythrozyten, die mit dem Fachmann bekannten Methoden, insbesondere mit Hilfe von Glutaraldehyd, fixiert und damit haltbarer gemacht werden. In einer bevorzugten Ausführungsform werden pro Testlauf verschiedene Indikatorpartikel verwendet, von denen mindestens eine Sorte native Erythrozyten sind.

In einer Ausführungsform der Erfindung sind die Indikatorzonen so angeordnet, dass die Probenflüssigkeit pro Fließspur nicht mehr als eine Indikatorzone durchströmt. Beispielhaft sind die Indikatorzonen versetzt auf der Membran angeordnet. Die Anordnung der Indikatorzonen ist dabei vorzugsweise in einer von proximal nach distal oder umgekehrt diagonal verlaufenden Reihe ausgestaltet. Besondere Ausführungsformen sind V-förmig, W-, M-, oder N-förmig oder umgekehrt V-förmig, W-, M-, oder N-förmig ausgestaltet. In einer weiteren Ausführungsform sind die Indikatorzonen parallel nebeneinander versetzt in einer linearen Reihe angeordnet.

Die Einführung parallel versetzter Indikatorzonen macht eine Multiparametertestung mit Erythrozyten als Indikatorpartikeln in einer lateralen Anordnung erst möglich. Weiterhin ist die Bestimmung von Analyten aus allen Bestandteilen von Vollblut möglich, d.h die gleichzeitige Bestimmung von zellulären und plasmatischen Parametern im gleichen Ansatz. Ferner wird dadurch die Testung von zellulären und plasmatischen Parametern aus einer Vollblutprobe im selben Ansatz unter Verwendung zweier verschiedener Sorten von Indikatorpartikeln möglich, von denen eine Sorte native Erythrozyten sind.

Die besonders bevorzugte Ausführungsform einer diagonalen Anordnung hat den Vorteil, dass die Bezeichnung der Ergebnisse besonders praktisch und leicht ablesbar auf die erfindungsgemäße Anordnung aufgebracht werden kann, da jeder nachzuweisende Parameter eine bestimmte X- und Y-Position aufweist, betrachtet man die Anordnung der erfindungsgemäßen Vorrichtung als ein Koordinatensystem mit Ordinate (Ebene der Fließrichtung) und Abszisse (Ebene der Auftragszone).

In einer weiteren Ausführungsform der Erfindung sind mehr als eine solche Reihe von Indikatorzonen, vorzugsweise in einer von proximal nach distal oder umgekehrt diagonal verlaufenden Reihe, oder beispielsweise in einer V-förmig, W-, M-, oder N-förmig oder umgekehrt V-förmig, W-, M-, oder N-förmig verlaufenden Reihe, oder beispielsweise auch in einer parallel nebeneinander versetzt verlaufenden Reihe, in Fließrichtung hintereinander angeordnet und die Indikatorzonen der verschiedenen Reihen entweder zueinander auf Lücke angeordnet, so dass die Probenflüssigkeit pro Fließspur nicht mehr als eine Indikatorzone durchströmt, oder zueinander nicht auf Lücke angeordnet, so dass die Probenflüssigkeit pro Fließspur mehr als eine Indikatorzone durchströmt.

Mehr als eine solche Reihe von Indikatorzonen, beispielsweise zwei Reihen von Indikatorzonen, mit unterschiedlicher Distanz zur Aufgabezone sind insbesondere dann vorteilhaft, wenn aus einer Vollblutprobe zelluläre und plasmatische Parameter bestimmt werden sollen. Erfindungsgemäß, beispielhaft in einem Testansatz mit Vollblut als Probe, werden die Bindungselemente so gewählt, dass die im Plasma enthaltenen Analyten, zum Beispiel jede Art von Antikörpern, die durch die Vorrichtung von der Aufgabezone zum Absorptionsbereich durch die Indikatorzonen fließen, in der proximal zur Aufgabezone angeordneten Reihe von Indikatorzonen an die Bindungselemente binden. Die Bindungselemente zum Nachweis der zellulär gebundenen Analyten, beispielsweise erythrozytäre Antigene, werden dagegen so gewählt, dass diese in der distal zur Aufgabezone angeordneten Reihe von Indikatorzonen an die Bindungselemente binden. Bei dieser erfindungsgemäßen Ausführungsform wird vorzugsweise mit einer zusätzlich zu den nativen Erythrozyten weiteren Sorte von Indikatorpartikeln, vorzugsweise aus kolloidalem Gold oder Polystyrol oder fixierte Erythrozyten, gearbeitet. Diese Indikatorpartikel werden insbesondere eingesetzt, um nicht erythrozytär gebundene Analyten, beispielsweise frei im Plasma vorkommende Antikörper, im Bindungskomplex in einer Indikatorzone sichtbar zu machen. Werden beispielhaft zwei Arten von Indikatorpartikeln verwendet, wovon eine nicht Erythrozyten sind, können die Indikatorzonen der beiden Reihen zueinander nicht auf Lücke bzw. in einer Fließspur hintereinander angeordnet sein. Vorteilhaft ist hierbei die Anordnung, bei der die aus dem Plasma nachgewiesenen Analyten in den proximalen Indikatorzonen nachgewiesen werden und bei der die Erythrozyten-gebundenen Analyten in den distalen Indikatorzonen nachgewiesen werden. In einer Ausführungsform der Erfindung mit mehr als einer, wie vorbeschriebenen Reihe von Indikatorzonen, von denen die Bindungselemente jeder Reihe mit Erythrozyten als Indikatorpartikel reagieren bzw. binden, sind die Reihen von Indikatorzonen zueinander auf Lücke bzw. in einer Fließspur nicht hintereinander angeordnet.

In einer weiteren Ausführungsform der Erfindung sind mehr als eine solche Reihe von Indikatorzonen, vorzugsweise in einer von proximal nach distal oder umgekehrt diagonal verlaufenden Reihe, oder beispielsweise in einer V-förmig, W-, M-, oder N-förmig oder umgekehrt V-förmig, W-, M-, oder N-förmig verlaufenden Reihe, oder beispielsweise auch in einer parallel nebeneinander versetzt verlaufenden Reihe, bidirektional zu einer zentralen Aufgabezone angeordnet. Eine solche erfindungsgemäße Anordnung ist insbesondere dann vorteilhaft, wenn aus einer Vollblutprobe zelluläre und plasmatische Parameter bestimmt werden.

Erfindugsgemäß, beispielhaft in einem Testansatz mit Vollblut als Probe, werden die Bindungselemente so gewählt, dass die im Plasma enthaltenen Analyten, zum Beispiel jede Art von Antikörpern, die durch die Vorrichtung von der Aufgabezone zum Absorptionsbereich durch die Indikatorzonen fließen, in der auf der einen Seite zur Aufgabezone angeordneten Reihe von Indikatorzonen an die Bindungselemente binden. Die Bindungselemente zum Nachweis der zellulär gebundenen Analyten, beispielsweise erythrozytäre Antigene, werden dagegen so gewählt, dass diese in der auf der gegenüberliegenden Seite zur Aufgabezone angeordneten Reihe von Indikatorzonen an die Bindungselemente binden. Bei dieser bevorzugten Ausführungsform wird vorzugsweise mit einer zusätzlich zu den Erythrozyten weiteren Sorte von Indikatorpartikeln, vorzugsweise aus kolloidalem Gold oder Polystyrol, gearbeitet. Diese Indikatorpartikel werden insbesondere eingesetzt, um nicht erythrozytär gebundene Analyten, beispielsweise frei im plasma vorkommende Antikörper, im Bindungskomplex in einer Indikatorzone sichtbar zu machen.

In einer bevorzugten Ausführungsform umfasst die eine Aufgabezone zudem zwei verschiedene Membranen unterschiedlicher Porosität. Bei dieser bevorzugten Ausführungsform wird vorzugsweise mit einer zusätzlich zu den Erythrozyten weiteren Sorte von Indikatorpartikeln, vorzugsweise aus kolloidalem Gold oder Polystyrol, gearbeitet. Diese Indikatorpartikel werden insbesondere eingesetzt, um nicht erythrozytär gebundene Analyten, beispielsweise frei im Plasma vorkommende Antikörper, im Bindungskomplex in einer Indikatorzone sichtbar zu machen.

In einer besonders bevorzugten Ausführungsform umfasst die eine Aufgabezone eine Membran oder zwei verschiedene Membranen unterschiedlicher Porosität. Ferner umfasst eine der beiden Membranen ein Konjugat-Pad, welches zwischen Dichtelement und Indikatorzonen angeordnet ist.. Bei dieser bevorzugten Ausführungsform wird vorzugsweise mit einer zusätzlich zu den Erythrozyten weiteren Sorte von Indikatorpartikeln, vorzugsweise aus kolloidalem Gold oder Polystyrol, gearbeitet. Diese Indikatorpartikel werden insbesondere eingesetzt, um nicht erythrozytär gebundene Analyten, beispielsweise frei im Plasma vorkommende Antikörper, im Bindungskomplex in einer Indikatorzone sichtbar zu machen. Diese weitere zusätzlich zu den Erythrozyten eingesetzte Sorte von Indikatorpartikeln liegt vorzugsweise in das Konjugat-Pad eingetrocknet vor. Weiterhin sorgt das Konjugat-Pad dafür, dass der Fluss der Erythrozyten verlangsamt wird. Dies führt dazu, dass in der bidirektionalen Anordnung bei einer einzigen gemeinsamen Aufgabezone in der einen Richtung optimierte Bedingungen für den Nachweis zellulärer Eigenschaften und in der anderen Richtung optimierte Bedingungen für den Nachweis plasmatischer Eigenschaften bereitgestellt werden.

Durch die erfindungsgemäße Vorrichtung wird ein Lateral-Fluss-Test, insbesondere für die blutgruppenserologische Diagnostik, bereitgestellt, mit dem Erythrozyten als Indikatorpartikel verwendet werden und in einem Testansatz gleichzeitig mehrere zelluläre, insbesondere erythrozytäre Antigene bzw. Antigen-Epitope, plasmatische Parameter und/oder Blutzelleigenschaften, insbesondere aus Vollblutbestandteilen, pro zu untersuchender Probe bestimmt werden können. Des weiteren wird damit ein möglichst einfach herzustellendes und einfach, insbesondere mit wenigen Versuchsreihen und ohne Probenvorbereitung, zu handhabendes und kostengünstiges Testsystem bereitgestellt, mit dem gleichzeitig verschiedene zelluläre Parameter und plasmatische Parameter einer Probe oder mehrerer zu untersuchender Proben, insbesondere Blutgruppenmerkmale, bestimmt werden können.

Diese Vorteile bietet die erfindungsgemäße Vorrichtung auf jedem medizinischdiagnostischen Gebiet, bei dem gleichzeitig verschiedene zelluläre Parameter und plasmatische Parameter bestimmt werden sollen, insbesondere auch auf dem Gebiet der Transfusionsmedizin, insbesondere der Blutgruppenserologie, und der Infektionsserologie, z. B. um Blutgruppenmerkmale, virale oder bakterielle Parameter simultan zu bestimmen, oder zur Bestimmung einer Blutgruppe in Kombination mit der Bestimmung von Antikörpern gegen Thrombozyten oder Lymphozyten oder zur Bestimmung einer Blutgruppe in Kombination mit der Serumgegenprobe. Hierzu kann antikoaguliertes oder natives Vollblut verwendet werden, bei dem vor der Bestimmung nicht in aufwendiger Weise Erythrozyten und Serum- bzw. Plasma-Fraktion von einander separiert werden müssen. Die Bestimmung kann in einem manuellen Format erfolgen, das komplett ohne Geräte (inklusive elektrischen Strom) auskommt.

Die Membran der erfindungsgemäßen Vorrichtung ist eine poröse Membran. Bevorzugte Membran-Materialien sind beispielsweise Nitrozellulose (z. B. *UniSart* (eingetragene Warenzeichen) von Sartorius, *HiFlow* (eingetragene Warenzeichen) von Millipore, Whatman, *AE99* bzw. *FF85*/*100* von Schleicher & Schuell), Polyethylen (*Lateral Flo* (eingetragene Warenzeichen) von Porex Corporation) oder Nylon (*Novylon* (eingetragene Warenzeichen) von CUNO). Vorzugsweise weist die Membran eine möglichst große Porengröße auf, da eine hohe Porosität der Membran das Eindringen insbesondere von zellulären Komponenten der zu bestimmenden Probe, z. B. von Erythrozyten, in die poröse Struktur begünstigt. Von besonderem Vorteil ist der Einsatz aufnehmender Membranen. Die erfindungsgemäße Vorrichtung ist jedoch nicht auf diese Eigenschaften beschränkt. Bevorzugt werden alle Membranen mit einer hohen kapillaren Flussrate (Capillary Speed), wobei die kapillare Flussrate die Zeit ist, die eine Farblösung braucht, um 40 mm auf einer gegebenen Membran zurückzulegen. Besonders bevorzugt sind Membranen, deren kapillare Flussrate < 100 ist.

In der bidirektionalen Ausführungsform mit zwei unterschiedlichen Membranen hat die eine Membran vorzugsweise eine hohe kapillare Flussrate, vorzugsweise < 100, die andere Membran vorzugsweise eine geringere kapillare Flussrate, vorzugsweise > 100.

In einer bevorzugten Ausführungsform der Erfindung ist in Fließrichtung hinter der Aufgabezone und vor den Indikatorzonen der erfindungsgemäßen Vorrichtung auf der porösen Membran ein Dichtelement angeordnet. Zur Anwendung kommen zwei- oder dreidimensionale Dichtelemente, die auf der porösen Membran platziert werden und mit denen eine von der übrigen Fläche der porösen Membran separierte Probenauftragszone geschaffen wird. Das Dichtelement hat erfindungsgemäß primär die Wirkung einer Flüssigkeitsbarriere und erlaubt die gerichtete Verteilung von Probenflüssigkeit und Testreagenzien in die poröse Membran. Weiterhin dichtet das Dichtelement erfindungsgemäß die Probenauftragszone ab zur Verhinderung eines unerwünschten Flüssigkeitsübertritts in die anderen Bereiche der Lateral-Fluss-Vorrichtungsanordnung.

Bevorzugte Ausführungsformen des Dichtelementes sind die Steg- oder Trog- bzw. Trichter-Form. Die Ausformung des Dichtelementes erfolgt durch Schneideprozesse aus dem zur Herstellung des Dichtelementes verwendeten Material. Im Fall der Trichter- bzw. Trogform erhält das Dichtelement eine innere Öffnung, deren bevorzugte Ausführungsvarianten runde, quadratische oder rechteckige, im Fall der Trichterform sich zur Unterseite (Membrankontaktseite) des Dichtelementes verjüngende Formen sind.

Bevorzugte Materialien für das Dichtelement sind Materialien, die nicht wasseraufnehmend (hydrophob) sind. In einer besonderen Ausführungsform sind die Materialien einseitig mit einem Klebstofffilm, beispielsweise einem drucksensitiven bzw. selbsthaftenden Acrylatklebstoff, beschichtet. Somit kann das Dichtelement direkt auf die Oberfläche der porösen Membran geklebt werden. Alternativ kann das Dichtelement mit dem Lateral-Fluss-Gehäuse verbunden, beispielsweise verklebt sein, wobei in dieser Ausführungsform das Lateral-Fluss-Gehäuse das Dichtelement auf die Oberfläche der porösen Membran drückt und damit die Funktionen des Dichtelementes erzielt werden.

Bevorzugte Materialien für die Ausbildung von zweidimensionalen Dichtelementen sind jede Form von Klebebändern oder Klebefolien (z. B. Tesa 4124 von Beiersdorf AG, ARcare 7815 von Adhesives Research).

Bevorzugte Materialien für die Ausbildung von dreidimensionalen Dichtelementen sind flexible, geschlossenporige Elastomermaterialien oder flexible Silikonmaterialien mit unterschiedlichen Materialstärken, vorzugsweise 3-5 mm (z. B. Zellkautschuk EPDM140 von Pitzner, Silikonkautschuk oder Vollkautschuk, Härte 40° oder weniger, von Castan).

Durch diese erfindungsgemäße Ausgestaltung ist die erfindungsgemäße Vorrichtung in der Lage, flüssige Proben, die Zellen enthalten, wie beispielsweise Vollblut, aufzunehmen, ohne die Zellen dabei abzufiltern. Weiterhin erlaubt das Dichtelement das Auftragen großer Probenvolumina auf die poröse Membran (Aufgabezone), ohne dass diese überschwemmt wird. Somit unterstützt das Dichtelement die Nutzung der aufnehmenden Eigenschaften der porösen Membran. Weiter garantiert das Dichtelement einen gerichteten Probenfluss. Die erfindungsgemäße Vorrichtung kann jedoch mit oder ohne Dichtelement gut funktionieren.

Für den Absorptionsbereich (Absorptions-Pad) der erfindungsgemäßen Vorrichtung werden mechanisch stabile Materialien bevorzugt, vorzugsweise mit Wasserabsorptionskapazitäten von 20-30 g/100 cm² (z. B. Wicking Papier, Typ 300, Schleicher und Schüll). Der Kontakt zwischen dem Absorptions-Pad und der Lateral Fluss-Membran der erfindungsgemäßen Vorrichtung wird durch Andruck und Überlappung mit der porösen Membran hergestellt. Die genaue Positionierung des Absorptions-Pads auf der Membran wird durch Verkleben des Absorptions-Pads mit der, die Lateral Fluss-Membran tragenden Trägerschicht (backing sheet), erzielt.

Das Konjugat-Pad besteht vorzugsweise aus Glasfaser oder Cellulose und hat vorzugsweise die Eigenschaft, den Fluss nativer Erythrozyten zu verzögern.

Die mit der erfindungsgemäßen Vorrichtung zu testenden Analyten befinden sich vorzugsweise in menschlichen oder tierischen Körperflüssigkeiten, beispielsweise im Urin, Blut, Serum, Plasma, Speichel, oder Bestandteilen davon, in therapeutischen Blut- und Plasmaprodukten, wie Eyrthrozytenkonzentraten, Thrombozytenkonzentraten oder frischgefrorenem Plasma, oder Komponenten davon (Probe/Proben). Die auf bestimmte Analyten hin zu untersuchende/untersuchenden Probe/Proben werden auf die Aufgabezone der erfindungsgemäßen Vorrichtung aufgetragen.

In einer weiteren Ausführungsform sind die Komponenten der erfindungsgemäßen Vorrichtung zum Zwecke der mechanischen Verstärkung auf eine Unterlage bzw. Trägerschicht aufgebracht. Die erfindungsgemäße Vorrichtung kann jedoch mit oder ohne Trägerschicht funktionieren. Bevorzugt werden mechanisch stabile und nicht wasseraufnehmende Materialien, vorzugsweise mit Materialstärken von 100 µm oder mehr, die ein- oder zweiseitig mit einem Klebstofffilm, z. B. einem drucksensitiven bzw. selbsthaftenden Acrylatklebstoff, beschichtet sind (z.B. 0.005" Polyester W/ GL-187, G & L). Auf der Trägerschicht werden die poröse Membran und das Absorptions-Pad fixiert. Im Fall der doppelseitig klebenden Trägerschicht wird die klebende zweite Seite zur Fixierung des Stapels auf weiteren Flächen, z. B. innerhalb der Lateral-Fluss-Gehäuse, eingesetzt.

In einer weiteren Ausführungsform ist die erfindungsgemäße Vorrichtung, entweder mit oder ohne Trägerschicht, auf der die Komponenten der erfindungsgemäßen Vorrichtung aufgebracht sind, in einem Gehäuse integriert, wodurch die Membran-Komponenten aneinander gedrückt werden und das Gehäuse die Dichtelementfunktion unterstützt. Dabei kann die erfindungsgemäße Vorrichtung jedoch mit oder ohne Gehäuse genauso gut funktionieren.

Ein weiterer Gegenstand der Erfindung ist die Verwendung der erfindungsgemäßen Vorrichtung zur Analyse von Körperflüssigkeiten von Menschen oder Tieren, insbesondere zur medizinischen Diagnostik, beispielsweise im Bereich der Blutgruppenserologie, der Mikrobiologie oder im Veterinärbereich, um vorzugsweise Krankheiten zu diagnostizieren, Therapieformen zu indizieren, Blutgruppenantigene bzw. -antikörper, die Anwesenheit bzw. Quantität von Arzneimitteln und/oder Drogen zu bestimmen. Bevorzugt verwendet werden dabei als Probe/Proben menschliche oder tierische Körper- bzw. Ausscheidungsflüssigkeiten, wie z. B. Urin, Saliva, Fäces, Peritonealflüssigkeit, Blut, Plasma, Serum, Blutzellen, Synovialflüssigkeit, Tränen-, Zerebrospinal-, Pankreas- oder Gallenflüssigkeit, therapeutische Blut- und Plasmaprodukte, wie Eyrthrozytenkonzentrate, Thrombozytenkonzentrate oder frischgefrorenes Plasma, oder Komponenten davon. Als weitere Proben kommen z. B. Trinkwasser, Schwimmbadwasser oder Erdproben zur Analyse in Betracht.

Im Folgenden werden Ausführungsbeispiele der Erfindung anhand der beigefügten Zeiclmungen erläutert. Es zeigen:
- Fig. 1: eine perspektivische Darstellung einer Vorrichtung für Lateral-Fluss-Tests für die simultane Bestimmung der Blutgruppenmerkmale A, B, AB, D und CDE;
- Fig. 2: eine Explosionsdarstellung der in Fig. 1 dargestellten Vorrichtung für Lateral-Fluss-Tests;
- Fig. 3: eine perspektivische Darstellung einer Vorrichtung für Lateral-Fluss-Tests für die simultane Bestimmung der Blutgruppenmerkmale A, B, AB, D und CDE, ausgeführt mit einem dreidimensionalen Dichtelement in Stegform;
- Fig. 4: eine Explosionsdarstellung der in Fig. 3 dargestellten Vorrichtung für Lateral-Fluss-Tests;
- Fig. 5: eine perspektivische Darstellung einer Vorrichtung für Lateral-Fluss-Tests für die simultane Bestimmung der Blutgruppenmerkmale A, B, AB, D und CDE, ausgeführt mit einem dreidimensionalen Dichtelement in Trogform;
- Fig. 6: eine Explosionsdarstellung der in Fig. 5 dargestellten Vorrichtung für Lateral-Fluss-Tests;
- Fig. 7: eine perspektivische Darstellung einer Vorrichtung für Lateral-Fluss-Tests für die simultane Bestimmung der Blutgruppenmerkmale A, B, AB, D, C, c, E, e, Cw und K;
- Fig. 8: eine perspektivische Darstellung einer Vorrichtung für Lateral-Fluss-Tests für die Durchführung des direkten Coombs-Tests;
- Fig. 9: eine perspektivische Darstellung einer erfindungsgemäßen Vorrichtung für Lateral-Fluss-Tests für die simultane Durchführung von Blutgruppenbestimmung, Serumgegenprobe und Antikörpersuchtest bei Empfängern;
- Fig. 10: eine perspektivische Darstellung einer erfindungsgemäßen Vorrichtung für Lateral-Fluss-Tests für die simultane Durchführung von Blutgruppenbestimmung, Serumgegenprobe und Antikörpersuchtest bei Spendern;
- Fig. 11: eine perspektivische Darstellung einer erfindungsgemäßen Vorrichtung für Lateral-Fluss-Tests für die simultane Durchführung von Blutgruppenbestimmung und dem Nachweis von Infektionskrankheitenspezifischen Antikörpern;
- Fig. 12: eine perspektivische Darstellung einer erfindungsgemäßen Vorrichtung für Lateral-Fluss-Tests für die simultane Durchführung von Blutgruppenbestimmung und dem Nachweis von Antikörpern gegen thrombozytäre Antigene.
- Fig. 13: eine perspektivische Darstellung einer Vorrichtung für Lateral-Fluss-Tests für die simultane Bestimmung der Blutgruppenmerkmale A, B, AB, D und CDE.
- Fig. 14: eine perspektivische Darstellung einer Vorrichtung für Lateral-Fluss-Tests für die simultane Bestimmung der Blutgruppenmerkmale A, B, AB, D und CDE.
- Fig. 15: eine perspektivische Darstellung einer Vorrichtung für Lateral-Fluss-Tests für die simultane Bestimmung der Blutgruppenmerkmale A, B, AB, D und CDE.
- Fig. 16: eine perspektivische Darstellung einer Vorrichtung für Lateral-Fluss-Tests für die simultane Bestimmung der Blutgruppenmerkmale A, B, AB, D und CDE.
- Fig. 17: zeigt eine perspektivische Darstellung einer erfindungsgemäßen Vorrichtung für Lateral-Fluss-Tests mit bidirektionalem Fluss für die simultane Bestimmung von Blutgruppenbestimmung und Serumgegenprobe;
- Fig. 18: zeigt eine Explosionsdarstellung der in Fig. 17 dargestellten erfindungsgemäßen Vorrichtung für Lateral-Fluss-Tests.
- Fig. 19: zeigt eine perspektivische Darstellung einer erfindungsgemäßen Vorrichtung für Lateral-Fluss-Tests mit bidirektionalem Fluss für die simultane Bestimmung von Blutgruppenbestimmung und Serumgegenprobe;
- Fig. 20: zeigt eine Explosionsdarstellung der in Fig. 17 dargestellten erfindungsgemäßen Vorrichtung für Lateral-Fluss-Tests.

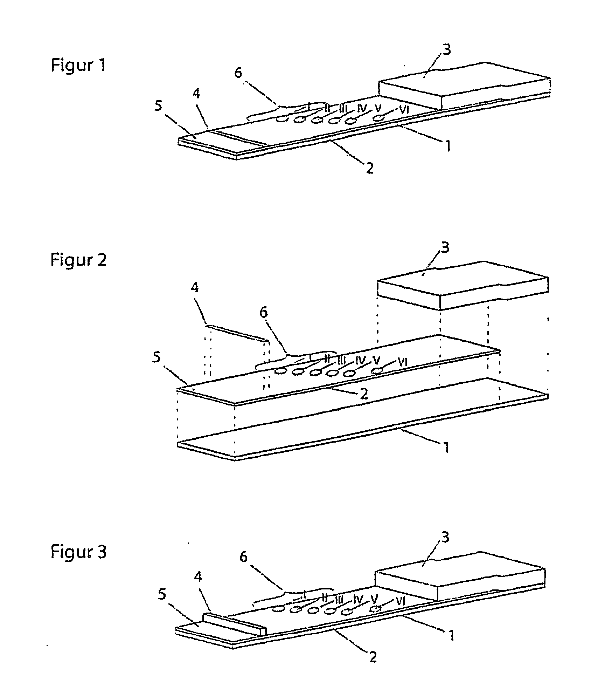

In Fig. 1 wird beispielhaft eine perspektivische Darstellung einer Vorrichtung für Lateral-Fluss-Tests für die simultane Bestimmung der Blutgruppenmerkmale A, B, AB, D und CDE gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I - VI gebildet, wobei die Indikatorzonen aus den folgenden Bindungselementen bestehen:

| Indikatorzone | Bindungselement | Spezifikation |
|---|---|---|
| | | |
| I | Antikörper | Anti-A (monoklonal) |
| II | Antikörper | Anti-B (monoklonal) |
| m | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen. In Fig. 2 wird eine Explosionsdarstellung der in Fig. 1 dargestellten Vorrichtung für Lateral-Fluss-Tests gezeigt, die aus den Komponenten Trägerschicht 1, poröse Membran 2, Absorptions-Pad 3 und Dichtelement 4 besteht, welches die Aufgabezone 5 von der übrigen Membran separiert, die wiederum den Indikatorzonenbereich 6 mit den von proximal nach distal diagonal versetzt angeordneten Indikatorzonen I-VI enthält.

In Fig. 3 wird beispielhaft eine perspektivische Darstellung einer Vorrichtung für Lateral-Fluss-Tests für die simultane Bestimmung der Blutgruppenmerkmale A, B, AB, D und CDE gezeigt. Im vorliegenden Beispiel entsprechen die Komponenten der Vorrichtung den Komponenten der in Fig. 1 dargestellten Vorrichtung mit Ausnahme des auf der Oberseite der porösen Membran 2 fixierten, in dreidimensionaler Stegform ausgeführten Dichtelements 4.

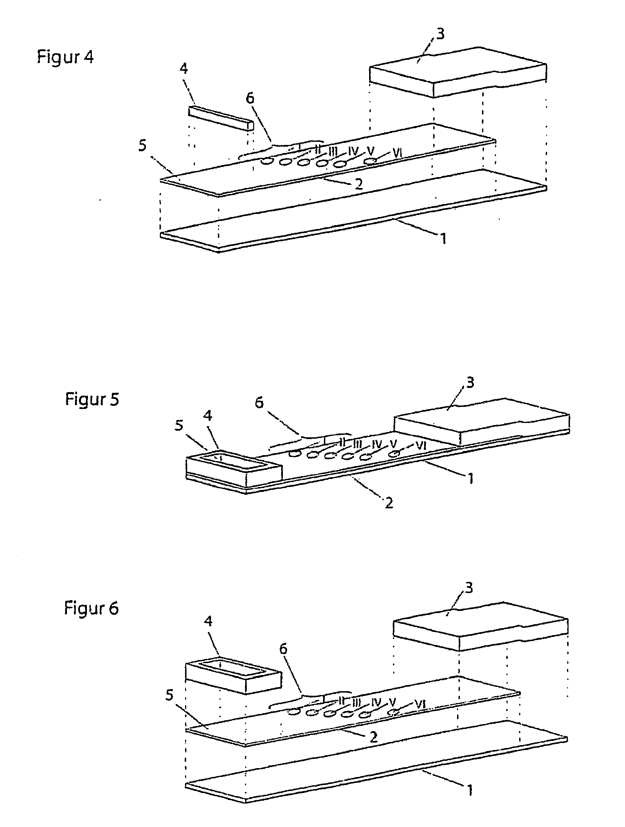

In Fig. 4 wird eine Explosionsdarstellung der in Fig. 3 dargestellten Vorrichtung für Lateral-Fluss-Tests mit den Komponenten Trägerschicht 1, poröse Membran 2, Absorptions-Pad 3 und in dreidimensionaler Stegform ausgeführtes Dichtelement 4 gezeigt, welches die Aufgabezone 5 von der übrigen Membran separiert, die wiederum den Indikatorzonenbereich 6 mit den von proximal nach distal diagonal versetzt angeordneten Indikatorzonen I-VI enthält.

In Fig. 5 wird beispielhaft eine perspektivische Darstellung einer Vorrichtung für Lateral-Fluss-Tests für die simultane Bestimmung der Blutgruppenmerkmale A, B, AB, D und CDE gezeigt. Im vorliegenden Beispiel entsprechen die Komponenten der Vorrichtung den Komponenten der in Fig. 1 dargestellten Vorrichtung mit Ausnahme des auf der Oberseite der porösen Membran 2 fixierten, in dreidimensionaler Trogform ausgeführten Dichtelements 4.

In Fig. 6 wird eine Explosionsdarstellung der in Fig. 5 dargestellten Vorrichtung für Lateral-Fluss-Tests mit den Komponenten Trägerschicht 1, poröse Membran 2, Absorptions-Pad 3 und in dreidimensionaler Trogform ausgeführtes Dichtelement 4 gezeigt, welches die Aufgabezone 5 von der übrigen Membran separiert, die wiederum den Indikatorzonenbereich 6 mit den von proximal nach distal diagonal versetzt angeordneten Indikatorzonen I-VI enthält.

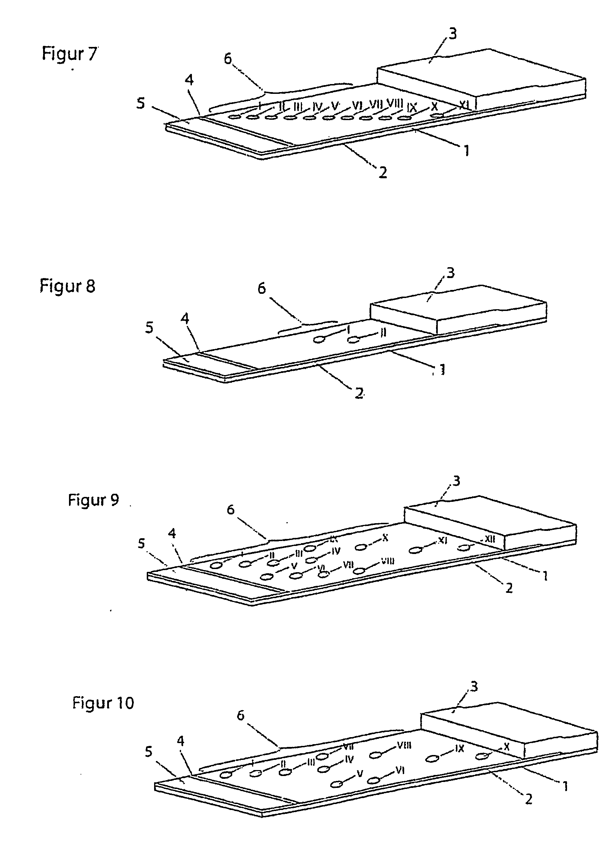

In Fig. 7 wird beispielhaft eine perspektivische Darstellung einer Vorrichtung für Lateral-Fluss-Tests für die simultane Bestimmung der Blutgruppenmerkmale A, B, AB, D, C, c, E, e, Cw und K gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I - XI gebildet, wobei die Indikatorzonen aus den folgenden Bindungselementen bestehen:

| Indikatorzone | Bindungselement | Spezifikation |
|---|---|---|
| | | |
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-C (monoklonal) |
| VI | Antikörper | Anti-c (monoklonal) |
| VII | Antikörper | Anti-E (monoklonal) |
| VIII | Antikörper | Anti-e (monoklonal) |
| IX | Antikörper | Anti-Cw (monoklonal) |
| X | Antikörper | Anti-K (monoklonal) |
| XI | Antikörper | Anti-Erythrozyten (polyklonal) |

Indikatorzone XI ist die Kontrolle (ctl) und enthält polyklonale anti-Erythrozyten Antikörper. Sie befindet sich distal angeordnet zu allen übrigen Indikatorzonen.

In Fig. 8 wird beispielhaft eine perspektivische Darstellung einer Vorrichtung für Lateral-Fluss-Tests für die Durchführung des direkten Coombs-Tests gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I und II gebildet, wobei die Indikatorzonen aus den folgenden Bindungselementen bestehen:

| Indikatorzone | Bindungselement | Spezifikation |
|---|---|---|
| | | |
| I | Antikörper | Anti-human IgG (polyklonal) |
| II | Antikörper | Anti-Erythrozyten (polyklonal) |

Indikatorzone II ist die Kontrolle (ct1) und enthält polyklonale anti-Erythrozyten Antikörper.

In Fig. 9 wird beispielhaft eine perspektivische Darstellung einer erfindungsgemäßen Vorrichtung für Lateral-Fluss-Tests für die simultane Durchführung von Blutgruppenbestimmung, Serumgegenprobe und Antikörpersuchtest bei Empfängern gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I-XII gebildet, wobei die Indikatorzonen I-VIII den Indikatorzonenbereich Serumgegenprobe/Antikörpersuchtest und die Indikatorzonen IX-XII den Indikatorzonenbereich zur Blutgruppenbestimmung umfassen und aus den folgenden Bindungselementen bestehen:

| Indikatorzone | Bindungselement | Spezifikation |
|---|---|---|
| | | |
| *Indikatorzonenbereich: Serumgegenprobe*/*Antikörpersuchtest* | | |
| | | |
| I | Erythrozyten-Ghosts | Blutgruppe A1 |
| n | Erythrozyten-Ghosts | Blutgruppe A2 |
| III | Erythrozyten-Ghosts | Blutgruppe B |
| IV | Erythrozyten-Ghosts | Blutgruppe 0 |
| V | Erythrozyten-Ghosts | Suchzelle 1, Blutgruppe 0, R1R1^{w} |
| VI | Erythrozyten-Ghosts | Suchzelle 2, Blutgruppe 0, R2R2 |
| VII | Erythrozyten-Ghosts | Suchzelle 3, Blutgruppe 0, rr |
| VIII | Antikörper | Anti-human IgG/IgM |
| | | |
| *Indikatorzonenbereich: Blutgruppenbestimmung* | | |
| | | |
| IX | Antikörper | Anti-A (monoklonal) |
| x | Antikörper | Anti-B (monoklonal) |
| XI | Antikörper | Anti-AB (monoklonal) |
| XII | Antikörper | Anti-Erythrazyten (polyklonal) |

Indikatorzone VIII ist die Kontrolle (ctl) für die Serumgegenprobe und Antikörpersuchtest und enthält anti-human IgG/IgM. Indikatorzone XII ist die Kontrolle (ct1) für die Blutgruppenbestimmung und enthält polyklonale anti-Erythrozyten Antikörper. Sie befindet sich distal angeordnet zu allen übrigen Indikatorzonen.

In Fig. 10 wird beispielhaft eine perspektivische Darstellung einer erfindungsgemäßen Vorrichtung für Lateral-Fluss-Tests für die simultane Durchführung von Blutgruppenbestimmung, Serumgegenprobe und Antikörpersuchtest bei Spendern gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen. Trägerschicht 1 fixierte. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4. separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I-X gebildet, wobei die Indikatorzonen I-VI den Indikatorzonenbereich Serumgegenprobe/Autikörpersuchtest und die Indikatorzonen VII-X den Indikatorzonenbereich zur Blutgruppenbestimmung umfassen und aus den folgenden Bindungselementen bestehen:

| Indikatorzone | Bindungselement | Spezifikation |
|---|---|---|
| | | |
| *Indikatorzonenbereich: Serumgegenprobe*/*Antikörpersuchtest* | | |
| | | |
| I | Erythrozyten-Ghosts | Blutgruppe A1 |
| II | Erythrozyten-Ghosts | Blutgruppe A2 |
| III | Erythrozyten-Ghosts | Blutgruppe B |
| IV | Erythrozyten-Ghosts | Blutgruppe 0 |
| V | Erythrozyten-Ghosts | Blutgruppe 0, Pool der Suchzellen 1, 2, 3 (siehe Beschreibung Fig. 9) |
| VI | Antikörper | Anti-human IgG/IgM |
| | | |
| *Indikatorzonenbereich: Blutgruppenbestimmung* | | |
| | | |
| VII | Antikörper | Anti-A (monoklonal) |
| VIII | Antikörper | Anti-B (monoklonal) |
| IX | Antikörper | Anti-AB (monoklonal) |
| X | Antikörper | Anti-Erythrozyten (polyklonal) |

Indikatorzone VI ist die Kontrolle (ctl) für die Serumgegenprobe und Antikörpersuchtest und enthält anti-human IgG/IgM. Indikatorzone X ist die Kontrolle (ctl) für die Blutgruppenbestimmung und enthält polyklonale anti-Erythrozyten Antikörper. Sie befindet sich distal angeordnet zu allen übrigen Indikatorzonen.

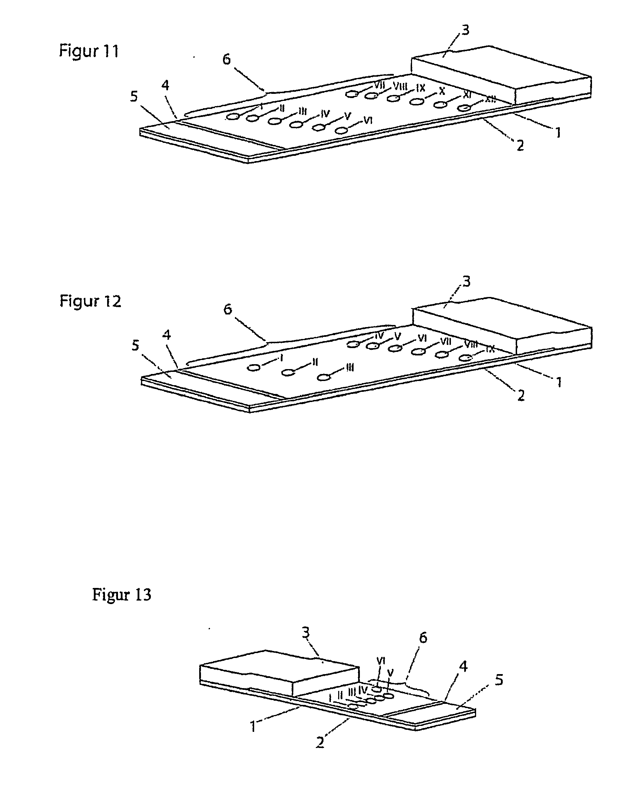

In Fig. 11 wird beispielhaft eine perspektivische Darstellung einer erfindungsgemäßen Vorrichtung für Lateral-Fluss-Tests für die simultane Durchführung von Blutgruppenbestimmung und Antikörpern gegen infektiöse Agenzien gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I-XII gebildet, wobei die Indikatorzonen I-VI den Indikatorzonenbereich zum Nachweis von Infektionsmarkern und die Indikatorzonen VII-XII den Indikatorzonenbereich zur Blutgruppenbestimmung umfassen und aus den folgenden Bindungselementen bestehen:

| Indikatorzone | Bindungselement | Spezifikation |
|---|---|---|
| | | |
| *Indikatorzonenbereich: Nachweis von Infektionsmarkern* | | |
| | | |
| I | Synthetische Peptide | HIV-I (gp-14, gp-42) |
| II | Synthetische Peptide | HIV-2 (gp-36) |
| III | Antikörper | Anti-HBsAg (monoklonal) |
| IV | Rekombinante Antigene | HCV (C-100, C-200, C33c, c22) |
| V | Rekombinantes Antigen | Syphilis (TpN 15, TpN 17, TpN 47) |
| VI | Antikörper | Anti-human IgG/IgM |
| | | |
| *Indikatorzonenbereich: Blutgruppenbestimmung* | | |
| | | |
| VII | Antikörper | Anti-A (monoklonal) |
| VIII | Antikörper | Anti-B (monoklonal) |
| IX | Antikörper | Anti-AB (monoklonal) |
| X | Antikörper | Anti-D (monoklonal) |
| XI | Antikörper | Anti-CDE (monoklonal) |
| XII | Antikörper | Anti-Erythrozyten (polyklonal) |

Indikatorzone VI ist die Kontrolle (ctl) für die Bestimmung von Antikörpern gegen infektiöse Agenzien und enthält anti-human IgG/IgM. Indikatorzone XII ist die Kontrolle (ctl) für die Blutgruppenbestimmung und enthält polyklonale anti-Erythrozyten Antikörper. Sie befindet sich distal angeordnet zu allen übrigen Indikatorzonen.

In Fig. 12 wird beispielhaft eine perspektivische Darstellung einer erfindungsgemäßen Vorrichtung für Lateral-Fluss-Tests für die simultane Durchführung von Blutgruppenbestimmung und dem Nachweis von Antikörpern gegen thrombozytäre Antigene, gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I-IX gebildet, wobei die Indikatorzone I-III den Indikatorzonenbereich zum Nachweis von Antikörpern gegen thrombozytäre Antigene und die Indikatorzonen IV-IX den Indikatorzonenbereich zur Blutgruppenbestimmung umfassen und aus den folgenden Bindungselementen bestehen:

| Indikatorzone | Bindungselement | Spezifikation |
|---|---|---|
| | | |
| *Indikatorzonenbereich: Nachweis von Antikörpern gegen thrombozytäre Antigene* | | |
| | | |
| I | Membranproteine | Thrombozylen, HPA 1bb3aa5bb |
| II | Membranproteine | Thrombozyten, HPA 1aa3bb5aa |
| III | Antikörper | Anti-human IgG/IgM |
| | | |
| | | |
| *Indikatorzonenbereich: Blutgruppenbestimmung* | | |
| | | |
| IV | Antikörper | Anti-A (1-monoklonal) |
| V | Antikörper | Anti-B (monoklonal) |
| VI | Antikörper | Anti-AB (monoklonal) |
| VII | Antikörper | Anti-D (monoklonal) |
| VIII | Antikörper | Anti-CDE (monoklonal) |
| IX | Antikörper | Anti-Erythrozyten (polyklonal) |

Indikatorzone III ist die Kontrolle (ctl) für den Nachweis von Antikörpern gegen thrombozytäre Antigene und enthält anti-human IgG/IgM. Indikatorzone IX ist die Kontrolle (ctl) für die Blutgruppenbestimmung und enthält polyklonale anti-Erythrozyten Antikörper. Sie befindet sich distal angeordnet zu allen übrigen Indikatorzonen.

In Fig. 13 wird beispielhaft eine perspektivische Darstellung einer Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Rechtshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus parallel nebeneinander versetzt, in einer linearen Reihe angeordneten, in definierten X- und Y-Positionen angeordneten, punktförmigen Indikatorzonen I - VI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

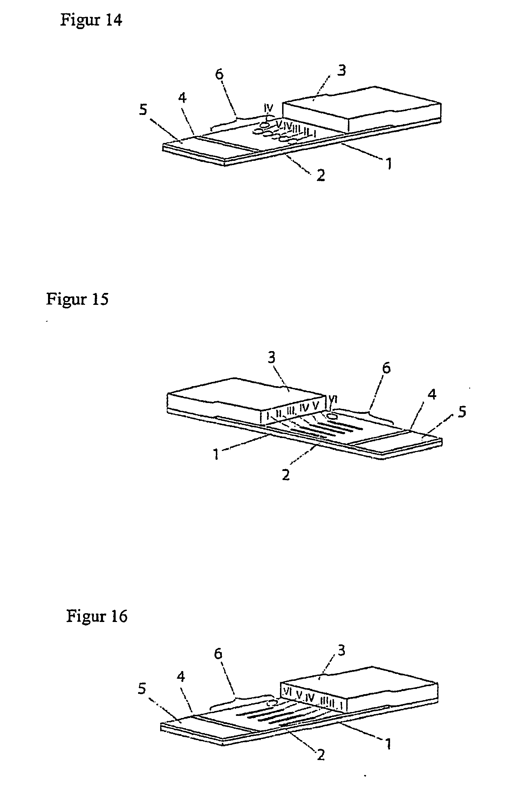

Indikatorzone VI ist die Kontrolle (ctl) und enthält polyklonale anti-Erythrozyten Antikörper. Sie befindet sich distal angeordnet zu allen übrigen Indikatorzonen. In Fig. 14 wird beispielhaft eine perspektivische Darstellung einer Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Linkshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatoronenbereich 6 angeordnet. Dieser wird aus parallel nebeneinander versetzt, in einer linearen Reihe angeordneten, in definierten X- und Y-Positionen angeordneten, punktförmigen Indikatorzonen I - VI gebildet, wobei die Indikatorzonen aus den folgenden Bindungselementen bestehen:

| Indikatorzone | Bindungselement | Spezifikation |
|---|---|---|
| | | |
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

In Fig. 15 wird beispielhaft eine perspektivische Darstellung einer Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Rechtshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus parallel nebeneinander versetzt angeordneten, in definierten X- und Y-Positionen angeordneten, längsgezogenen bzw. bandförmigen Indikatorzonen I - VI gebildet, wobei die Indikatorzonen aus den folgenden Bindungselementen bestehen:

| Indikatorzone | Bindungselement | Spezifikation |
|---|---|---|
| | | |
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

In Fig. 16 wird beispielhaft eine perspektivische Darstellung einer Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Linkshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus parallel nebeneinander versetzt angeordneten, in definierten X- und Y-Positionen angeordneten, längsgezogenen bzw. bandförmigen Indikatorzonen I - VI gebildet, wobei die Indikatorzonen aus den folgenden Bindungselementen bestehen:

| Indikatorzone | Bindungselement | Spezifikation |
|---|---|---|
| | | |
| I | Antikörper | Anti-A (monoklonal) |
| n | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

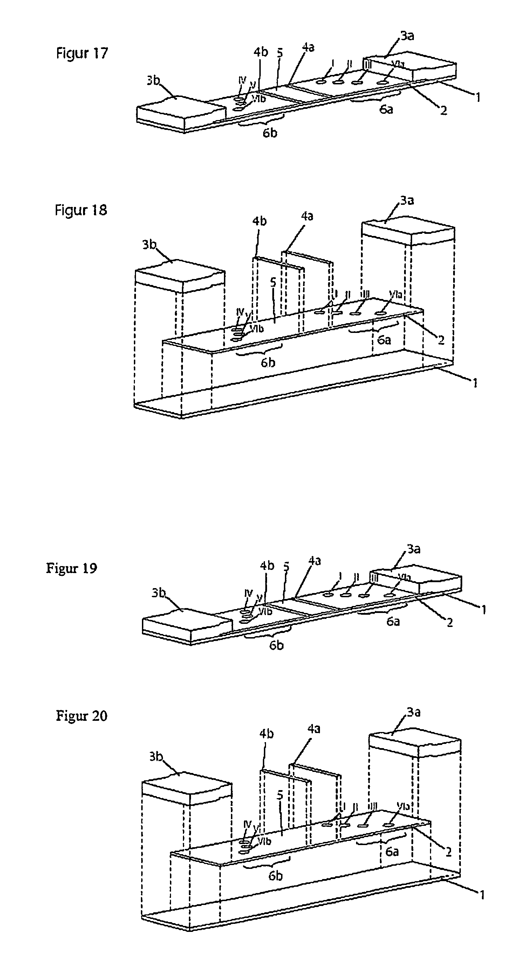

In Fig. 17 wird beispielhaft eine perspektivische Darstellung einer erfindungsgemäßen Vorrichtung für Lateral-Fluss-Tests mit bidirektionalem Fluss für die simultane Bestimmung von Blutgruppenbestimmung und Serumgegenprobe gezeigt. Im vorliegenden Beispiel besteht die Vorrichtung aus einer Trägerschicht 1, einer porösen Membran 2a für die Blutgruppenbestimmung, einer von Membran 2a sich unterscheidenden porösen Membran 2b für die Serumgegenprobe, den Absorptions-Pads 3 a und 3b, einem dreidimensionalen, in Trogform ausgeführten Dichtelement 4, und einem Konjugat-Pad 6. Dabei sind die porösen Membranen 2a und 2b auf der mit einem drucksensitiven Acrylatklebstoff versehenen Trägerschicht 1 fixiert. Ebenso sind die Absorptions-Pads 3a und 3b auf der Trägerschicht 1 fixiert, wobei je ein Teil der Absorptions-Pads 3a und 3b mit den porösen Membranen 2a und 2b überlappen. Das auf der Oberseite der porösen Membran 2a und 2b fixierte Dichtelement 4 separiert die Aufgabezonen 5a bzw. 5b von den übrigen Membranflächen und ermöglicht die gerichtete Verteilung von Probenflüssigkeit und Testreagenzien in die porösen Membranen 2a und 2b. Zwischen der Aufgabezone 5a und dem Bereich der porösen Membran 2a, der mit dem Absorptions-Pad 3a in Kontakt steht, ist der Indikatorzonenbereich 7a (Blutgruppenbestimmung) angeordnet. Dieser wird aus diagonal versetzten, in definierten X- und Y-Positionen angeordneten, punktförmigen Indikatorzonen I - VI gebildet, wobei die Indikatorzonen des Indikatorzonenbereiches 7a aus den folgenden Bindungselementen bestehen:

| Indikatorzone | Bindungselement | Spezifikation |
|---|---|---|
| | | |
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Die Indikatorzone VI ist die Kontrolle (ctl) für die Blutgruppenbestimmung und enthält polyklonale anti-Erythrozyten Antikörper. Sie befindet sich distal angeordnet zu den Indikatorzonen I-V.

Zwischen der Aufgabezone 5b und dem Bereich der porösen Membran 2b, der mit dem Absorptions-Pad 3b in Kontakt steht, ist der Indikatorzonenbereich 7b (Serumgegenprobe) angeordnet. Dieser wird aus diagonal versetzten, in definierten X- und Y-Positionen angeordneten, punktförmigen Indikatorzonen VII - IX gebildet, wobei die Indikatorzonen des Indikatorzonenbereiches 7b aus den folgenden Bindungselementen bestehen:

| Indikatorzone | Bindungselement | Spezifikation |
|---|---|---|
| | | |
| VII | Erythrozyten-Ghosts | Blutgruppe A |
| VIII | Erythrozyten-Ghosts | Blutgruppe B |
| IX | Erythrozyten-Ghosts | Blutgruppe 0 (Kontrolle) - |

In Fig. 18 wird eine Explosionsdarstellung der in Fig. 17 dargestellten erfindungsgemäßen Vorrichtung für Lateral-Fluss-Tests mit bidirektionalem Fluss gezeigt, die aus den Komponenten Trägerschicht 1, einer porösen Membran 2a für die Blutgruppenbestimmung, einer von Membran 2a sich unterscheidenden porösen Membran 2b für die Serumgegenprobe, den Absorptions-Pads 3a und 3b, einem dreidimensionalen, in Trogform ausgeführten Dichtelement 4, und einem Konjugat-Pad 6 bestehen. Die Probenaufgabezone erstreckt sich über beide porösen Membranen mit der Probenaufgabezone 5a der Membran 2a und der Probenaufgabezone 5b der Membran 2b und wird durch das in Trogform ausgeführte Dichtelement 4 von den übrigen Flächen der Membranen 2a und 2b separiert. Die Membran 2a enthält den Indikatorzonenbereich 7a mit den von proximal nach distal diagonal versetzt angeordneten Indikatorzonen I-VI, während die Membran 2b den Indikatorzonenbereich 7b mit den von proximal nach distal diagonal versetzt angeordneten Indikatorzonen VII-LX enthält.

In Fig. 19 wird beispielhaft eine perspektivische Darstellung einer erfindungsgemäßen Vorrichtung für Lateral-Fluss-Tests mit bidirektionalem Fluss für die simultane Bestimmung von Blutgruppenbestimmung und Serumgegenprobe gezeigt. Im vorliegenden Beispiel besteht die Vorrichtung aus einer Trägerschicht 1, einer porösen Membran 2a für die Blutgruppenbestimmung, einer von Membran 2a sich unterscheidenden porösen Membran 2b für die Serumgegenprobe, den Absorptions-Pads 3a und 3b, einem dreidimensionalen, in Trogform ausgeführten Dichtelement 4, und einem Konjugat-Pad 6. Dabei sind die porösen Membranen 2a und 2b auf der mit einem drucksensitiven Acrylatklebstoff versehenen Trägerschicht 1 fixiert. Ebenso sind die Absorptions-Pads 3a und 3b auf der Trägerschicht 1 fixiert, wobei je ein Teil der Absorptions-Pads 3a und 3b mit den porösen Membranen 2a und 2b überlappen. Das auf der Oberseite der porösen Membran 2a und 2b fixierte Dichtelement 4 separiert die Aufgabezonen 5a bzw. 5b von den übrigen Membranflächen und ermöglicht die gerichtete Verteilung von Probenflüssigkeit und Testreagenzien in die porösen Membranen 2a und 2b. Zwischen der Aufgabezone 5a und dem Bereich der porösen Membran 2a, der mit dem Absorptions-Pad 3a in Kontakt steht, ist der Indikatorzonenbereich 7a (Blutgruppenbestimmung) angeordnet. Dieser wird aus diagonal versetzten, in definierten X- und Y-Positionen angeordneten, punktförmigen Indikatorzonen I- VI gebildet, wobei die Indikatorzonen des Indikatorzonenbereiches 7a aus den folgenden Bindungselementen bestehen:

| Indikatorzone | Bindungselement | Spezifikation |
|---|---|---|
| | | |
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Die Indikatorzone VI ist die Kontrolle (ctl) für die Blutgruppenbestimmung und enthält polyklonale anti-Erythrozyten Antikörper. Sie befindet sich distal angeordnet zu den Indikatorzonen I-V.

Zwischen der Aufgabezone 5b und dem Bereich der porösen Membran 2b, der mit dem Absorptions-Pad 3b in Kontakt steht, ist der Indikatorzonenbereich 7b (Serumgegenprobe) angeordnet. Dieser wird aus diagonal versetzten, in definierten X- und Y-Positionen angeordneten, punktförmigen Indikatorzonen VII - IX gebildet, wobei die Indikatorzonen des Indikatorzonenbereiches 7b aus den folgenden Bindungselementen bestehen:

| Indikatorzone | Bindungselement | Spezifikation |
|---|---|---|
| | | |
| VII | Erythrozyten-Ghosts | Blutgruppe A |
| VIII | Erythrozyten-Ghosts | Blutgruppe B |
| IX | Erythrozyten-Ghosts | Blutgruppe 0 (Kontrolle) |

In Fig. 20 wird eine Explosionsdarstellung der in Fig. 19 dargestellten erfindungsgemäßen Vorrichtung für Lateral-Fluss-Tests mit bidirektionalem Fluss gezeigt, die aus den Komponenten Trägerschicht 1, einer porösen Membran 2a für die Blutgruppenbestimmung, einer von Membran 2a sich unterscheidenden porösen Membran 2b für die Serumgegenprobe, den Absorptions-Pads 3a und 3b, einem dreidimensionalen, in Trogform ausgeführten Dichtelement 4, und einem Konjugat-Pad 6 bestehen. Die Probenaufgabezone erstreckt sich über beide porösen Membranen mit der Probenaufgabezone 5a der Membran 2a und der Probenaufgabezone 5b der Membran 2b und wird durch das in Trogform ausgeführte Dichtelement 4 von den übrigen Flächen der Membranen 2a und 2b separiert. Die Membran 2a enthält den Indikatorzonenbereich 7a mit den von proximal nach distal diagonal versetzt angeordneten Indikatorzonen I-VI, während die Membran 2b den Indikatorzonenbereich 7b mit den von proximal nach distal diagonal versetzt angeordneten Indikatorzonen VII-IX enthält.

## Patentansprüche

1. Vorrichtung zum gleichzeitigen und qualitativen oder quantitativen Bestimmen mehrerer zellulär-gebundener und plasmatischer Analyten, wobei wenigstens ein Analyt ein zellulär-gebundener Analyt ist, in einer flüssigen Probe, umfassend mindestens eine Membran (2) mit
- einer Aufgabezone (5) zum Auftragen der flüssigen Probe,
- mindestens einer Gruppe von mindestens zwei Indikatorzonen, die mit den Analyten in Wechselwirkung treten können und
- mindestens einem Absorptionsbereich (3), welcher die Flüssigkeit nach Passieren der Indikatorzonen aufnimmt, wobei die Indikatorzonen zwischen der Aufgabezone (5) und einem Absorptionsbereich (3) liegen, **dadurch gekennzeichnet, dass** die Fließrichtungen von der Aufgabezone (5) durch die jeweiligen Indikatorzonen einer Gruppe zu einem Absorptionsbereich (3) (Fließspuren) im Wesentlichen parallel sind und mindestens zwei unterschiedliche Fließspuren vorliegen und
sofern die Indikatorzonen mit mehr als einem zellulär-gebundenen Analyten reagieren, die Indikatorzonen für diese Analyten nicht hintereinander angeordnet sind und
wobei die Indikatorzonen so angeordnet sind, dass die Indikatorzone des zellulär-gebundenen Analyten distal zur Aufgabezone und die Indikatorzone des plasmatischen Analyten proximal zur Aufgabezone angeordnet sind.

2. Vorrichtung nach Anspruch 1, wobei die Indikatorzonen so angeordnet sind, dass die Probenflüssigkeit pro Fließspur nicht mehr als eine Indikatorzone durchströmt.

3. Vorrichtung nach Anspruch 1, wobei die Indikatorzonen in einer diagonalen, V-, W-, M-, oder N-förmigen oder linearen Reihe angeordnet sind.

4. Vorrichtung nach einem der Ansprüche 1 bis 3, wobei die Indikatorzonen Antikörper bzw. Antikörperfragmente, Lektine, Antigene bzw. Antigen-Epitope und/oder Zellen bzw. Zellfragmente umfassen.

5. Vorrichtung nach einem der Ansprüche 1 bis 4, wobei die Indikatorzonen insbesondere anti-A, -B, -AB, -D,, -C, -c, -E, -e, -Cw, und/oder -K-Antikörper bzw. Antikörperfragmente umfassen

6. Vorrichtung nach einem der Ansprüche 1 bis 5, wobei die Membran oder die Membranen(2) vorzugsweise aus Polyethylen, Nitrozellulose oder Nylon, besteht.

7. Vorrichtung nach einem der Ansprüche 1 bis 6, wobei hinter der Aufgabezone (5) und vor den Indikatorzonen auf der Membran oder den Membranen (2) mindestens ein Dichtelement (4) angeordnet ist.

8. Vorrichtung nach einem der Ansprüche 1 bis 7, wobei die Aufgabezone und die Indikatorzonen zwei unterschiedliche Membranen umfassen.

9. Vorrichtung nach Anspruch 8, wobei bei einer der zwei Membranen hinter dem Dichtelement und vor den Indikatorzonen ein Konjugat-Pad aufgebracht ist.

10. Vorrichtung nach einem der Ansprüche 1 bis 9, wobei die Komponenten der Vorrichtung zur mechanischen Verstärkung auf eine Trägerschicht (1) aufgebracht sind.

11. Vorrichtung nach einem der Ansprüche 1 bis 10, wobei die Komponenten der Vorrichtung in einem Gehäuse integriert sind.

12. Verwendung der Vorrichtung nach einem der Ansprüche 1 bis 11 zur Analyse von Blut, Blutplasma, Blutzellen, Serum, Ham oder Saliva oder Bestandteilen davon.

## Claims

1. Device for the simultaneous and qualitative or quantitative determination of a plurality of cellularly bound or plasmatic analytes in a liquid sample, wherein at least one analyte is a cellularly bound analyte, comprising at least one membrane (2) with
- an application zone (5) for the application of the liquid sample,
- at least one group of at least two indicator zones, which are able to interact with the analytes and
- at least one absorption region (3), which takes up the liquid after passage of the indicator zones,
wherein the indicator zones are located between the application zone (5) and an absorption region (3), **characterized in that**
the flow directions from the application zone (5) through the respective indicator zone of a group towards an absorption region (3) (flow tracks) are substantially parallel and at least two different flow tacks are present and
in case the indicator zones react with more than one cellularly bound analyte, the indicator zones for these analytes are not arranged behind one another and
wherein the indicator zones are arranged such that the indicator zone of the cellularly bound analyte is arranged distally to the application zone and the indicator zone of the plasmatic analyte is arranged proximally to the application zone.

2. Device according to claim 1, wherein the indicator zones are arranged such that the sample liquid does not flow through more than one indicator zone per flow track.

3. Device according to claim 1, wherein the indicator zones are arranged in a diagonal, V-, W-, M- or N-shaped series or in a linear series.

4. Device according to any one of claims 1 to 3, wherein the indicator zones comprise antibodies respectively antibody fragments, lectines, antigens respectively antigen epitopes, and/or cells respectively cell fragments.

5. Device according to any one of claims 1 to 4, wherein the indicator zones comprise in particular anti-A, -B, -AB, -D, -C, -c, -E, -e, -Cw, and/or -K antibodies respectively antibody fragments.

6. Device according to any one of claims 1 to 5, wherein the membrane or the membranes (2) consist preferably of polyethylene, nitrocellulose or nylon.

7. Device according to any one of claims 1 to 6, wherein downstream of the application zone (5) and upstream of the indicator zones at least one sealing element (4) is arranged on the membrane or the membranes (2).

8. Device according to any one of claims 1 to 7, wherein the application zone and the indicator zone comprise two different membranes.

9. Device according to claim 8, wherein a conjugation pad is applied on one of the two membranes downstream of the sealing element and upstream of the indicator zones.

10. Device according to any one of claims i to 9, wherein the components of the device are applied onto a support layer (1) for mechanical reinforcement.

11. Device according to any one of claims 1 to 10, wherein the components of the device are integrated in a casing.

12. Use of the device according to any one of claims 1 to 11 for the analysis of blood, blood plasma, serum, urine or saliva or constituents thereof.

## Revendications

1. Dispositif pour la détermination simultanée et qualitative ou quantitative de plusieurs analytes plasmatiques et à liaison cellulaire, au moins un analyte étant un analyte à liaison cellulaire, dans un échantillon liquide, comprenant au moins une membrane (2), qui comporte
- une zone de dépôt (5) pour application de l'échantillon liquide,
- au moins un groupe d'au moins deux zones indicatrices, qui peuvent entrer en interaction avec les analytes, et
- au moins un domaine d'absorption (3), qui absorbe le liquide après passage par les zones indicatrices,
les zones indicatrices se trouvant entre la zone de dépôt (5) et un domaine d'absorption 3, **caractérisé en ce que** les directions d'écoulement, à partir de la zone de dépôt (5), en passant par les zones indicatrices respectives d'un groupe, pour arriver à un domaine d'absorption (3) (traces d'écoulement) sont pour l'essentiel parallèles, et au moins deux traces d'écoulement différentes sont présentes, et, dans la mesure où les zones indicatrices réagissent avec plus d'un analyte à liaison cellulaire, les zones indicatrices pour ces analytes ne sont pas disposées l'une derrière l'autre ; et les zones indicatrices étant disposées de telle sorte que la zone indicatrice de l'analyte à liaison cellulaire soit disposée en position distale par rapport à la zone de dépôt, et que la zone indicatrice de l'analyte plasmatique soit disposée en position proximale par rapport à la zone de dépôt.

2. Dispositif selon la revendication 1, dans lequel les zones indicatrices sont disposées de telle sorte que le liquide échantillon ne traverse pas plus d'une zone indicatrice par trace d'écoulement.

3. Dispositif selon la revendication 1, dans lequel les zones indicatrices sont disposées en une série diagonale, en forme de V, de W, de M ou de N, ou linéaire.

4. Dispositif selon l'une des revendications 1 à 3, dans lequel les zones indicatrices comprennent des anticorps ou des fragments d'anticorps, des lectines, des antigènes ou des épitopes d'antigènes, et/ou des cellules ou des fragments de cellules.

5. Dispositif selon l'une des revendications 1 à 4, dans lequel les zones indicatrices comprennent en particulier des anticorps ou des fragments d'anticorps anti-A, -B, -AB, -D, -C, -c, -E, -e, Cw et/ou K.

6. Dispositif selon l'une des revendications 1 à 5, dans lequel la membrane ou les membranes (2) sont constituées de préférence de polyéthylène, de nitrocellulose ou de nylon.

7. Dispositif selon l'une des revendications 1 à 6, dans lequel, en arrière de la zone de dépôt (5) et en avant des zones indicatrices, au moins un élément étanche (4) est disposé sur la membrane ou les membranes (2).

8. Dispositif selon l'une des revendications 1 à 7, dans lequel la zone de dépôt et les zones indicatrices comprennent deux membranes différentes.

9. Dispositif selon la revendication 8, dans lequel un tampon conjugué est appliqué, sur l'une des deux membranes, en arrière de l'élément étanche et en avant des zones indicatrices.

10. Dispositif selon l'une des revendications 1 à 9, dans lequel les composants du dispositif sont, pour un renforcement mécanique, disposés sur une couche support (1).

11. Dispositif selon l'une des revendications 1 à 10, dans lequel les composants du dispositif sont intégrés dans un boîtier.

12. Utilisation du dispositif selon l'une des revendications 1 à 11 pour l'analyse du sang, du plasma sanguin, des cellules sanguines, du sérum, de l'urine ou de la salive, ou de constituants de ceux-ci.
